# EUROPEAN PATENT SPECIFICATION

(11) **EP 2 668 925 B1**
(45) Date of publication and mention of the grant of the patent: **19.10.2016**
(21) Application number: 12169718.9
(22) Date of filing: 29.05.2012
(51) Int. Cl.: A61C 8/00, A61B 17/86, A61C 13/00

(54) **A serrated dental implant**
Gezacktes Zahnimplantat
Implant dentaire crénelé

(43) Date of publication of application: 04.12.2013
(73) Proprietor: A.B. Dental Devices Ltd., 79245 Nir Galim (IL)
(72) Inventor: Bar Shalom, Eliezer, 79245 Nir Galim (IL)
(74) Representative: Pearl Cohen Zedek Latzer Baratz UK LLP

(56) References cited:
- EP-A2- 2 215 990
- WO-A1-2011/039162
- ES-A1- 2 203 305
- JP-A- 2008 245 994
- KR-B1- 100 912 272

## Description

### BACKGROUND

### 1. TECHNICAL FIELD

The present invention relates to the field of dentistry, and more particularly, to a dental implant.

### 2. DISCUSSION OF RELATED ART

The following documents illustrate various prior art dental implants, in which the outer thread is serrated in order to increase the surface area of the thread and stabilize implant in the bone: US7273373, US6679701, US6386877, US5110245, WO2011039162, WO2007074498 and JP8019555 disclose variants of a uniform horizontal serration, which differ in the exact form of the saw-teeth formed on the outer thread.

### BRIEF SUMMARY

One aspect of the present invention provides a dental implant comprising (i) an outer thread that is serrated to have saw-teeth, each saw-tooth having a vertical inclination in a proximal-distal direction, an extent in a radial direction in respect to an axis of the implant, and a horizontal inclination tangential to a radius extending from the axis of the implant, and (ii) at least one body thread on a body of the implant that winds parallel to the outer thread, wherein the at least one body thread is serrated to have saw-teeth, each saw-tooth having a vertical inclination in a proximal-distal direction, an extent in a radial direction in respect to the axis of the implant, and a horizontal inclination tangential to a radius extending from the axis of the implant, wherein the horizontal inclination of the saw-teeth of the at least one body thread is in an opposite direction in respect to the saw-teeth of the horizontal inclination of the outer thread.

### BRIEF DESCRIPTION OF THE DRAWINGS

For a better understanding of embodiments of the invention and to show how the same may be carried into effect, reference will now be made, purely by way of example, to the accompanying drawings in which like numerals designate corresponding elements or sections throughout.

In the accompanying drawings:
**Figures 1****,** **2A****,** **2B** and **3** are schematic illustrations of a dental implant according to some embodiments of the invention, and
**Figure 4** is a high level flowchart illustrating a method of producing or designing a dental implant, to some embodiments of the invention.

### DETAILED DESCRIPTION

With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

Before explaining at least one embodiment of the invention in detail, it is to be understood that the invention is not limited in its application to the details of construction and the arrangement of the components set forth in the following description or illustrated in the drawings. The invention is applicable to other embodiments or of being practiced or carried out in various ways. Also, it is to be understood that the phraseology and terminology employed herein is for the purpose of description and should not be regarded as limiting.

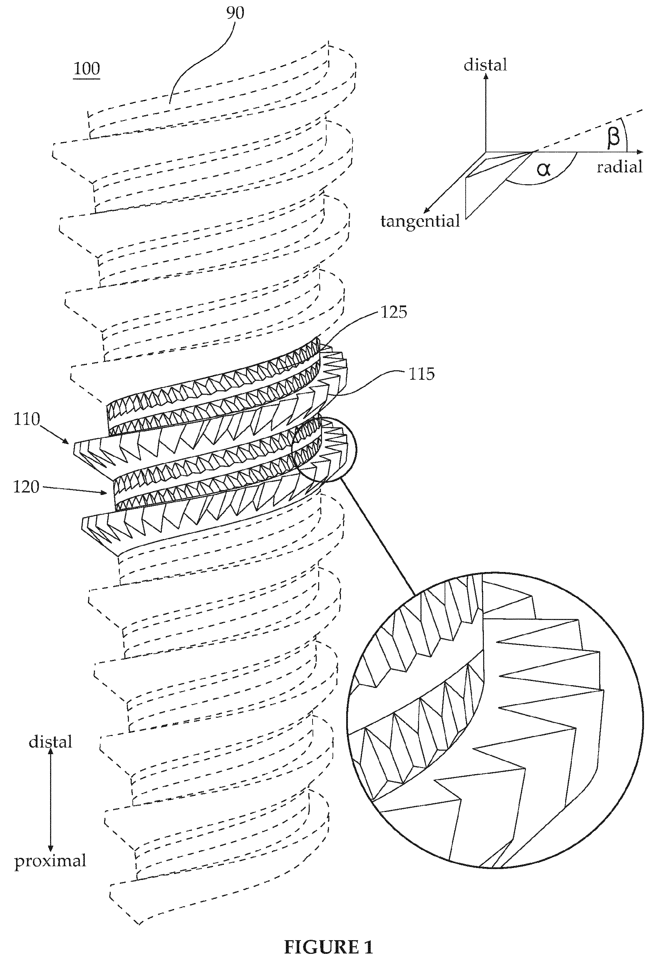

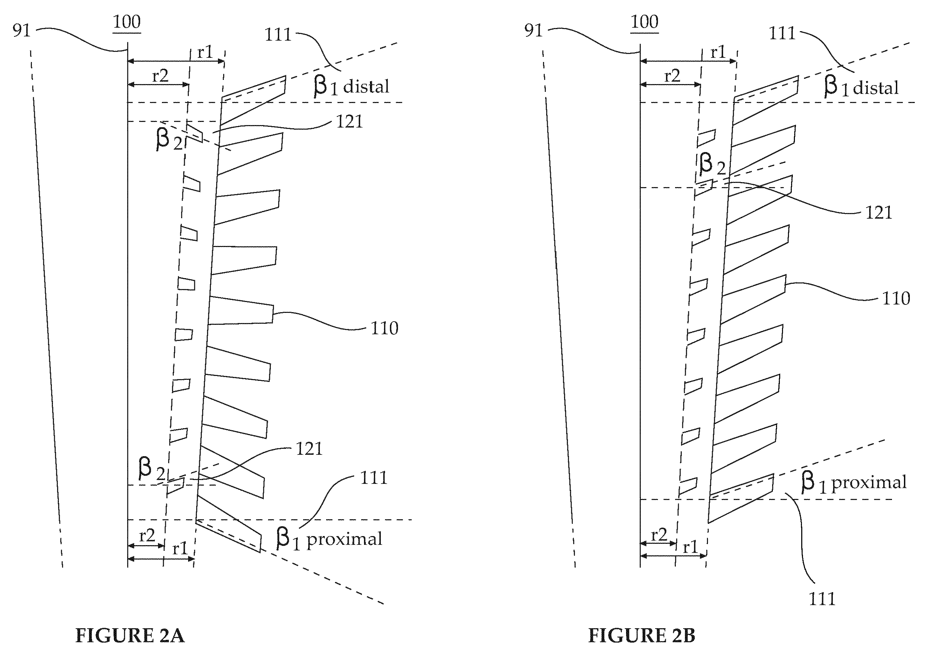

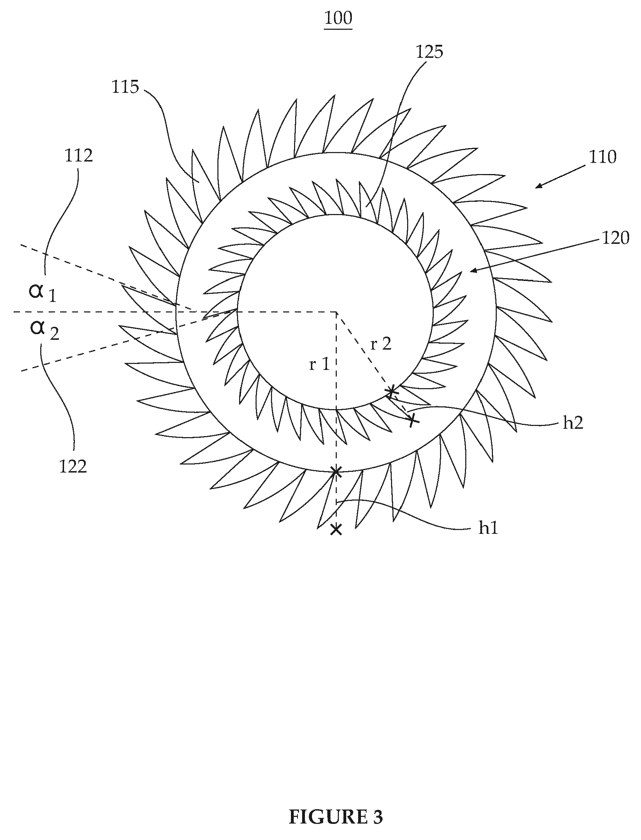

**Figures 1****,** **2A, 2B** and **C** are schematic illustrations of a dental implant **100** according to some embodiments of the invention. Dental implant **100** has a serrated outer thread **110** and body threads **120** serrated in the opposite direction to outer thread **110.** The serration of outer thread **110** improves the penetration of implant **100** into the jawbone, while body threads **120** improve the anchoring of implant **100** within the jaw bone. The insets in **Figure 1** illustrate the spatial relations of the saw-teeth **115** and **125.** **Figures 2A** and **2B** are high level schematic illustrations of a longitudinal section through implant **100**, illustrating schematically two possible configurations of serrated outer thread **110.** **Figure 3** is a high level schematic illustration of a transverse section through implant **100,** illustrating schematically the relative orientations of serrated outer thread **110** and serrated body thread **120.**

Dental implant **100** comprises outer thread **110** that is serrated to have saw-teeth **115**, each saw-tooth **115** having a vertical inclination β₁ **111** in a proximal-distal direction, an extent h₁ in a radial direction in respect to an axis **91** of implant **100**, and a horizontal inclination α₁ **112** tangential to a radius r₁ extending from axis **91** of implant **100.**

Dental implant **100** comprises at least one body thread **120** on a body **90** of implant **100** that winds parallel to outer thread **110**, wherein at least one body thread **120** is serrated to have saw-teeth **125**, each saw-tooth **125** having a vertical inclination β₂, **121** in a proximal-distal direction, an extent h₂ in a radial direction in respect to axis **91** of implant **100**, and a horizontal inclination α₂, **122** tangential to a radius r₂ extending from axis **91** of implant **100.**

Horizontal inclination **122** of the saw-teeth of the at least one body thread **120** is in an opposite direction in respect to the saw-teeth of the horizontal inclination **112** of the outer thread **110.**

Implant **100** may be produced by laser sintering techniques to generate the intricate structure disclosed. Implants with different spatial parameters may be designed and produced simultaneously by this technique.

Examples for possible embodiments of implant **100** may include the following.

Vertical inclination **111** (β₁) of outer saw-teeth **115** may be between 0° and 10° distad (i.e. in distal direction).

Vertical inclination **121** (β₂) of inner saw-teeth **115** may be around 0°, or a few degrees above or below the level.

Horizontal inclination **112** (α₁) of outer saw-teeth **115** may be between 0° and 10° clockwise from the radius (r₁).

Horizontal inclination **122** (α) of outer saw-teeth **125** may be between 0° and 5° counterclockwise from the radius (r₂).

Horizontal inclination **112** (α₁) of outer saw-teeth **115** may be clockwise from the radius (r₁) and horizontal inclination **122** (α₂) of inner saw-teeth **125** may be counterclockwise to the radius (r₂).

The extent (h₁) of outer saw-teeth **115** may be between 0.4 and 1 mm. For example, for implants **100** having a diameter of 3.75 mm outer saw-teeth **115** may protrude by about 0.4 mm on each side, while in larger implants **100** having diameters between 4.5-6 outer saw-teeth **115** may protrude by up to 1 mm on each side. In embodiments, outer saw-teeth **115** may extend by 15-35% of the implant's radius (h₁ = 0.15 to 0.35 times r₁ (or times (r₁+h₁)).

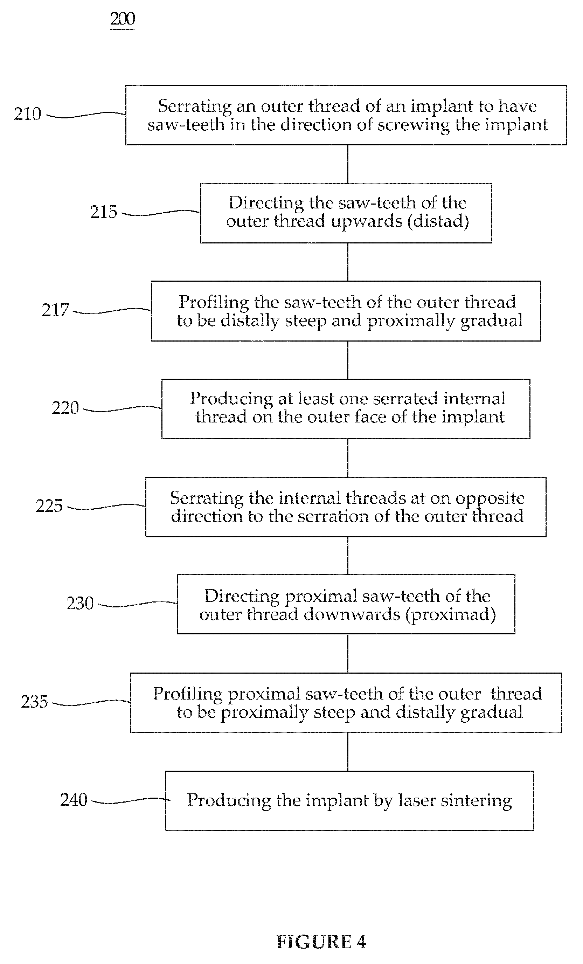

**Figure 4** is a high level flowchart illustrating a method **200** of producing or designing a dental implant, to some embodiments of the invention.

Method **200** comprises at least some of the following stage: serrating an outer thread of an implant to have saw-teeth at the direction of screwing the implant (stage 210), directing the saw-teeth of the outer thread upwards distad (stage **215**), profiling the saw-teeth of the outer thread to be distally steep and proximally gradual (stage **217**), producing at least one serrated internal thread on the outer face of the implant (stage **220**), serrating the internal threads at an opposite direction to the serration of the outer thread (stage **225**), for example, directing the outer saw-teeth clockwise and directing he inner saw-teeth counterclockwise, directing proximal saw-teeth of the outer thread downwards proximad (stage **230**), and profiling proximal saw-teeth of the outer thread to be proximally steep and distally gradual (stage **235**). Method **200** may be carried out by laser sintering (stage **240**) to allow the exact production of the intricate structure.

In the above description, an embodiment is an example or implementation of the invention. The various appearances of "one embodiment", "an embodiment" or "some embodiments" do not necessarily all refer to the same embodiments.

Although various features of the invention may be described in the context of a single embodiment, the features may also be provided separately or in any suitable combination. Conversely, although the invention may be described herein in the context of separate embodiments for clarity, the invention may also be implemented in a single embodiment.

Embodiments of the invention may include features from different embodiments disclosed above, and embodiments may incorporate elements from other embodiments disclosed above. The disclosure of elements of the invention in the context of a specific embodiment is not to be taken as limiting their used in the specific embodiment alone.

Furthermore, it is to be understood that the invention can be carried out or practiced in various ways and that the invention can be implemented in embodiments other than the ones outlined in the description above.

The invention is not limited to those diagrams or to the corresponding descriptions. For example, flow need not move through each illustrated box or state, or in exactly the same order as illustrated and described.

Meanings of technical and scientific terms used herein are to be commonly understood as by one of ordinary skill in the art to which the invention belongs, unless otherwise defined.

While the invention has been described with respect to a limited number of embodiments, these should not be construed as limitations on the scope of the invention, but rather as exemplifications of some of the preferred embodiments. Other possible variations, modifications, and applications are also within the scope of the invention.

## Claims

1. A dental implant (100) comprising:
an outer thread (110) that is serrated to have saw-teeth (115), each saw-tooth (115) having a vertical inclination (111, β₁) in a proximal-distal direction, as illustrated in figures 2A and 2B an extent (h₁) in a radial direction in respect to an axis (91) of the implant (100), and a horizontal inclination (112, α₁) tangential to a radius (r₁) extending from the axis (91) of the implant (100) as illustrated in figure 3, and
at least one body thread (120) on a body (90) of the implant (100) that winds parallel to the outer thread (110), wherein the at least one body thread (120) is serrated to have saw-teeth (125), each saw-tooth (125) having a vertical inclination (121, β₂) in a proximal-distal direction, as illustrated in figures 2A and 2B an extent (h₂) in a radial direction in respect to the axis (91) of the implant (100), and a horizontal inclination (122, α₂) tangential to a radius (r₂) extending from the axis (91) of as illustrated in figure 3,
wherein the horizontal inclination (122) of the saw-teeth of the at least one body thread (120) is in an opposite direction in respect to the saw-teeth of the horizontal inclination (112) of the outer thread (110).

2. The dental implant of claim 1, wherein the implant (100) is produced by laser sintering.

3. The dental implant of claim 1, wherein the vertical inclination (111, β₁) of the saw-teeth (115) of the outer thread is between 0° and 10° distad.

4. The dental implant of claim 1, wherein the vertical inclination (121, β₂) of the saw-teeth (125) of the body thread is substantially 0°.

5. The dental implant of claim 1, wherein the horizontal inclination (112, α₁) of the saw-teeth (115) of the outer thread is between 0° and 10° clockwise from the radius (r₁).

6. The dental implant of claim 1, wherein the horizontal inclination (122, α₂) of the saw-teeth (125) of the body thread is between 0° and 5° counterclockwise from the radius (r₂).

7. The dental implant of claim 1, wherein the horizontal inclination (112, α₁) of the saw-teeth (115) of the outer thread is clockwise from the radius (r₁) and the horizontal inclination (122, α₂) of the saw-teeth (125) of the body thread counterclockwise to the radius (r₂).

8. The dental implant of claim 1, wherein the extent (h₁) of the saw-teeth (115) of the outer thread is between 0.4 and 1 mm.

9. A method comprising
serrating an outer thread of an implant to have saw-teeth at the direction of screwing the implant,
directing the saw-teeth of the outer thread in a distal direction,
profiling the saw-teeth of the outer thread to be distally steep and proximally gradual, producing at least one serrated body thread on the outer face of the implant,
serrating the internal threads at an opposite direction to the serration of the outer thread, and profiling proximal saw-teeth of the outer thread to be proximally steep and distally gradual.
thereby producing a dental implant according to any of the claims 1-8

10. The method of claim 9, further comprising directing proximal saw-teeth of the outer thread in a proximal direction.

11. The method of claim 9, carried out by laser sintering.

12. The method of claim 9, wherein the saw-teeth of the outer thread are directed clockwise and the saw-teeth of the body thread are directed counterclockwise.

## Patentansprüche

1. Zahnimplantat (100), umfassend:
ein Außengewinde (110), das gezackt ist, so dass es Sägezähne (115) aufweist, wobei jeder Sägezahn (115) eine vertikale Neigung (111, β₁) in einer proximal-distalen-Richtung aufweist, wie in den Figuren 2A und 2B dargestellt;
eine Erstreckung (h₁) in einer radialen Richtung in Bezug auf eine Achse (91) des Implantats (100) und eine horizontale Neigung (112, α₁) tangential zu einem Radius (r₁), der von der Achse (91) des Implantats (100) ausgeht, wie in Figur 3 dargestellt ist, und
mindestens ein Körpergewinde (120) an einem Körper (90) des Implantats (100), das parallel zum Außengewinde (110) dreht, wobei das mindestens eine Körpergewinde (120) gezackt ist, so dass es Sägezähne (125) aufweist, wobei jeder Sägezahn (125) eine vertikale Neigung (121, β₂) in einer proximal-distalen Richtung aufweist, wie in den Figuren 2A und 2B dargestellt ist;
eine Erstreckung (h₂) in einer radialen Richtung mit Bezug auf die Achse (91) des Implantats (100) und eine horizontale Neigung (122, α₂) tangential zu einem Radius (r₂), der von der Achse (91) ausgeht, wie in Figur 3 dargestellt,
wobei die horizontale Neigung (122) der Sägezähne des mindestens einen Körpergewindes (120) entgegengesetzt ist zu den Sägezähnen mit der horizontalen Neigung (112) des Außengewindes (110).

2. Zahnimplantat nach Anspruch 1, wobei das Implantat (100) durch Lasersintern erzeugt ist.

3. Zahnimplantat nach Anspruch 1, wobei die vertikale Neigung (111, β₁) der Sägezähne (115) des Außengewindes zwischen 0° und 10° distal liegt.

4. Zahnimplantat nach Anspruch 1, wobei die vertikale Neigung (121, β₂) der Sägezähne (125) des Körpergewindes im Wesentlichen 0° beträgt.

5. Zahnimplantat nach Anspruch 1, wobei die horizontale Neigung (112, α₁) der Sägezähne (115) des Außengewindes zwischen 0° und 10° im Uhrzeigersinn vom Radius (r₁) aus beträgt.

6. Zahnimplantat nach Anspruch 1, wobei die horizontale Neigung (122, α₂) der Sägezähne (125) des Körpergewindes zwischen 0° und 5° entgegen dem Uhrzeigersinn vom Radius aus (r₂) beträgt.

7. Zahnimplantat nach Anspruch 1, wobei die horizontale Neigung (112, α₁) der Sägezähne (115) des Außengewindes im Uhrzeigersinn vom Radius (r₁) ausgeht und die horizontale Neigung (122, α₂) der Sägezähne (125) des Körpergewindes entgegengesetzt zum Uhrzeigersinn zum Radius (r₂) ist.

8. Zahnimplantat nach Anspruch 1, wobei das Erstreckung (h₁) der Sägezähne (115) des Außengewindes zwischen 0,4 und 1 mm beträgt.

9. Verfahren, umfassend:
Auszacken eines Außengewindes eines Implantats, so dass dieses Sägezähne in der Richtung aufweist, in der das Implantat eingeschraubt wird,
Ausrichten der Sägezähne des Außengewindes in einer distalen Richtung,
Profilieren der Sägezähne des Außengewindes, so dass sie distal steil und proximal graduell sind,
Erzeugen mindestens eines gezackten Körpergewindes an der Außenfläche des Implantats,
Auszacken der Innengewinde in einer entgegengesetzten Richtung zu den Zacken des Außengewindes, und
Profilieren der Sägezähne des Außengewindes, so dass sie proximal steil und distal graduell sind,
wodurch ein Zahnimplantat nach einem der Ansprüche 1-8 produziert wird.

10. Verfahren nach Anspruch 9, ferner das Ausrichten proximaler Sägezähne des Außengewindes in einer proximalen Richtung umfassend.

11. Verfahren nach Anspruch 9, das durch Lasersintern ausgeführt wird.

12. Verfahren nach Anspruch 9, wobei die Sägezähne des Außengewindes im Uhrzeigersinn ausgerichtet werden und die Sägezähne des Körpergewindes entgegen dem Uhrzeigersinn ausgerichtet werden.

## Revendications

1. Un implant dentaire (100) comprenant :
un filetage externe (110) qui est crénelé pour présenter des dents de scie (115), chaque dent de scie (115) présentant une inclinaison verticale (111, β₁) dans une direction proximale distale, comme illustrée dans les figures 2A et 2B ;
une extension (h₁) dans une direction radiale par rapport à un axe (91) de l'implant (100) et une inclinaison horizontale (112, α₁) tangente à un rayon (r₁) s'étendant à partir de l'axe (91) de l'implant (100), comme illustré à la figure 3, et
au moins un filetage du corps (120) sur un corps (90) de l'implant (100) qui s'enroule parallèlement au filetage externe (110), dans lequel au moins un filetage du corps (120) est crénelé pour présenter des dents de scie (125), chaque dent de scie (125) présentant une inclinaison verticale (121, β₂) dans la direction proximale distale, comme illustrée dans les figures 2A et 2B ;
une extension (h₂) dans une direction radiale par rapport à l'axe (91) de l'implant (100) et une inclinaison horizontale (122, α₂) tangente à un rayon (r₂) s'étendant à partir de l'axe (91), comme illustré à la figure 3,
dans lequel l'inclinaison horizontale (122) des dents de scie de l'au moins un filetage du corps (120) est dans une direction opposée par rapport aux dents de scie de l'inclinaison horizontale (112) du filetage externe (110).

2. L'implant dentaire selon la revendication 1, dans laquelle l'implant (100) est réalisé par frittage laser.

3. L'implant dentaire selon la revendication 1, dans laquelle l'inclinaison verticale (111, β₁) des dents de scie (115) du filetage externe est comprise entre 0° et 10° distalement.

4. L'implant dentaire selon la revendication 1, dans laquelle l'inclinaison verticale (121, β₂) des dents de scie (125) du filetage du corps est pratiquement 0°.

5. L'implant dentaire selon la revendication 1, dans laquelle l'inclinaison verticale (112, α₁) des dents de scie (115) du filetage externe est comprise entre 0° et 10° dans le sens horaire à partir du rayon (r₁).

6. L'implant dentaire selon la revendication 1, dans laquelle l'inclinaison verticale (122, α₂) des dents de scie (125) du filetage du corps est comprise entre 0° et 5° dans le sens antihoraire à partir du rayon (r₂).

7. L'implant dentaire selon la revendication 1, dans laquelle l'inclinaison verticale (112, α₁) des dents de scie (115) du filetage externe est dans le sens horaire à partir du rayon (r₁) et l'inclinaison horizontale (122, α₂) des dents de scie (125) du filetage du corps dans le sens antihoraire au rayon (r₂).

8. L'implant dentaire selon la revendication 1, dans laquelle l'extension (h₁) des dents de scie (115) du filetage externe est comprise entre 0,4 et 1 mm.

9. Un procédé comprenant
La dentelure d'un filetage externe d'un implant pour présenter des dents de scie dans la direction de vissage de l'implant,
La direction des dents de scie du filetage externe dans une direction distale,
le profilage des dents de scie du filetage externe pour être distalement raide et proximalement progressif,
la production d'au moins un filetage du corps crénelé sur la face externe de l'implant,
la dentelure des filetages internes dans une direction opposée à la denture du filetage externe, et
le profilage des dents de scie proximales du filetage externe pour être distalement raide et distalement progressif,
ce qui permet la production d'un implant dentaire selon une des revendications 1 à 8.

10. Le procédé de la revendication 9, comprenant en outre la direction des dents de scie proximales du filetage externe dans la direction proximale.

11. Le procédé de la revendication 9, réalisé par frittage laser.

12. Le procédé de la revendication 9, dans laquelle les dents de scie du filetage externe sont dirigées dans le sens horaire et les dents de scie du filetage du corps sont dirigées dans le sens antihoraire.
